# FASCICULE DE BREVET EUROPEEN

(11) **EP 2 951 189 B1**
(45) Date de publication et mention de la délivrance du brevet: **14.12.2016**
(21) Numéro de dépôt: 14701203.3
(22) Date de dépôt: 24.01.2014
(51) Int. Cl.: C07H 17/08, A61K 31/70, A61P 31/00

(54) **MACROLIDES UTILES COMME AGENTS ANTICANCEREUX**
MAKROLIDE ALS ANTIKREBSMITTEL
MACROLIDES USEFUL AS ANTICANCER AGENTS

(30) Priorité: 24.01.2013 FR 1350616
(43) Date de publication de la demande: 09.12.2015
(73) Titulaire: Pierre Fabre Médicament, 92100 Boulogne-Billancourt (FR); Centre National de la Recherche Scientifique (C.N.R.S.), 75016 Paris (FR); Institut De Recherche Pour Le Développement (IRD), 13000 Marseille (FR)
(72) Inventeur: CARLETTI, Isabelle, F-31400 Toulouse (FR); MASSIOT, Georges, F-51350 Cormontreuil (FR)
(74) Mandataire: Regimbeau
(86) Numéro de dépôt international: PCT/EP2014/051361
(87) Numéro de publication internationale: WO 2014/114729

(56) Documents cités:
- FAULKNER D J: "MARINE NATURAL PRODUCTS", NATURAL PRODUCT REPORTS, ROYAL SOCIETY OF CHEMISTRY. LONDON, GB, vol. 18, no. 1, 1 janvier 2001 (2001-01-01), pages 1-49, XP009056684,
- FAULKNER D J: "MARINE NATURAL PRODUCTS", NATURAL PRODUCT REPORTS, ROYAL SOCIETY OF CHEMISTRY. LONDON, GB, 1 janvier 1996 (1996-01-01), pages 113-158, XP000917606,
- FAULKNER D J: "MARINE NATURAL PRODUCTS", NATURAL PRODUCT REPORTS, XX, XX, vol. 19, 1 janvier 2002 (2002-01-01), pages 1-48, XP001068397,

## Description

La présente invention concerne des molécules de type macrolide extraites d'un spongiaire et ses analogues, un procédé d'extraction et d'hémi-synthèse et l'utilisation de ces composés en tant que médicament, notamment pour le traitement du cancer.

Depuis quelques dizaines d'années, les éponges marines sont devenues le centre de nombreuses études depuis la mise en évidence de leur production de métabolites secondaires bioactifs, en particulier les alcaloïdes (cf. par ex.D. John Faulkner Nat. Prod. Rep. 2001,18,1-49 ou Nat. Prod. Rep. 2002, 19,1-48).

Les inventeurs ont ainsi pu isoler une nouvelle famille de molécules à partir de l'éponge de la famille *Verongidae* telle qu'une éponge *Suberea sp.,* par exemple *Suberea creba,* provenant de Nouvelle Calédonie, qui présente une activité anticancéreuse.

La présente invention a donc pour objet un composé de formule (I) suivante : 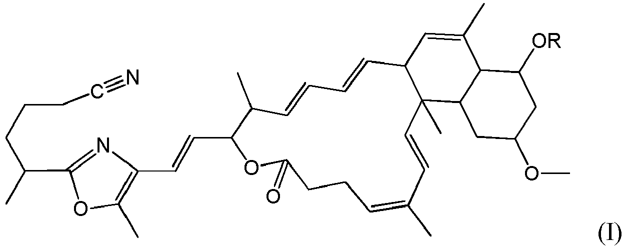 ou un sel, hydrate ou solvate pharmaceutiquement acceptable de celui-ci, dans laquelle R représente un résidu de monosaccharide ou disaccharide, les fonctions hydroxyles du résidu de monosaccharide ou disaccharide étant, indépendamment les unes des autres, éventuellement substituées par un groupe (C₁-C₆)alkyle, de préférence méthyle, ou -C(O)NH-(C₁-C₆)alkyle, de préférence -C(O)NHMe.

Dans la présente invention, on entend désigner par « pharmaceutiquement acceptable » ce qui est utile dans la préparation d'une composition pharmaceutique qui est généralement sûre, non toxique et ni biologiquement ni autrement non souhaitable et qui est acceptable pour une utilisation vétérinaire de même que pharmaceutique humaine.

On entend désigner par « sel, hydrate ou solvate pharmaceutiquement acceptable » d'un composé, un sel, hydrate ou solvate qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, et
(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Par groupement « (C₁-C₆)alkyle », on entend, au sens de la présente invention, une chaîne hydrocarbonée monovalente saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle. De préférence il s'agira d'un groupe méthyle.

Par « monosaccharide », on entend, au sens de la présente invention, un aldose (saccharide portant une fonction aldéhyde en position terminale, c'est-à-dire sur l'atome de carbone 1), et plus particulièrement un aldohexose (saccharide à 6 atomes de carbone) ou un aldopentose (saccharide à 5 atomes de carbone), de préférence un aldohexose. Il s'agira donc en particulier de l'allose, de l'altrose, du glucose, du mannose, du gulose, de l'idose, du galactose, du talose, du ribose, de l'arabinose, du xylose ou du lyxose, sous forme D ou L. Le monosaccharide sera de préférence sous une forme cyclisée, en particulier pyranique (cycle à 6 chaînons). Dans ce cas, la fonction aldéhyde portée par le saccharide se trouve sous une forme hémi-acétal, aussi appelée fonction pseudo-aldéhyde.

Par « disaccharide », on entend, au sens de la présente invention, une molécule résultant de la condensation de deux monosaccharides tels que définis ci-dessus, une telle condensation s'accompagnant de la perte d'une molécule d'eau. Cette réaction de condensation s'effectue dans le cadre de la présente invention entre la fonction aldéhyde (ou pseudo-aldéhyde) de l'un des monosaccharides, et une fonction hydroxy (OH) de l'autre monosaccharide. Le disaccharide résultant possède donc une et une seule fonction aldéhyde (ou pseudo-aldéhyde). De préférence, le disaccharide résultera de la condensation de la fonction aldéhyde (ou pseudo-aldéhyde) d'un aldopentose et d'une fonction hydroxy (OH) d'un aldohexose.

Par « résidu de monosaccharide ou disaccharide », on entend, au sens de la présente invention, la partie d'un monosaccharide ou disaccharide, tel que défini ci-dessus, qui est liée au reste de la molécule par l'intermédiaire de son atome de carbone 1 suite à une réaction de condensation entre la fonction aldéhyde (ou pseudo-aldéhyde) du monosaccharide ou disaccharide et une fonction hydroxy (OH).

Dans le cas d'un résidu d'un monosaccharide de type aldohexose sous forme pyranique, il s'agira donc du groupe suivant :

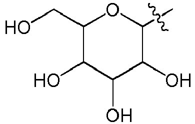

De préférence, le groupe R sera le groupe suivant : 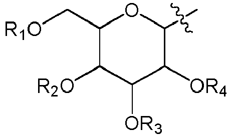 dans lequel les groupes R₁ à R₄ représentent, indépendamment les uns des autres, un atome d'hydrogène, un groupe (C₁-C₆)alkyle, de préférence méthyle, un groupe -C(O)NH-(C₁-C₆)alkyle, de préférence -C(O)NHMe, ou un résidu de monosaccharide de formule suivante : 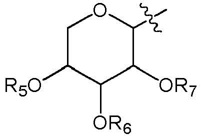 dans lequel les groupes R₅ à R₇ représentent, indépendamment les uns des autres, un atome d'hydrogène, un groupe (C₁-C₆)alkyle, de préférence méthyle, ou un groupe -C(O)NH-(C₁-C₆)alkyle, de préférence -C(O)NHMe,
un seul groupe R₁ à R₄ pouvant représenter un résidu de monosaccharide.

Avantageusement, R₁ = R₄ = Me, R₂ = H et R₃ = H ou un résidu de monosaccharide de formule suivante :

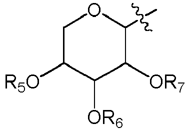

Le groupe R pourra être en particulier un groupe : 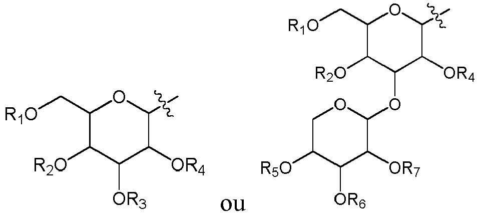 dans lequel :
- R₁ à R₄ représentent, indépendamment les uns des autres, un atome d'hydrogène, un groupe (C₁-C₆)alkyle, de préférence méthyle, ou un groupe -C(O)NH-(C₁-C₆)alkyle, de préférence -C(O)NHMe ; notamment un atome d'hydrogène ou un groupe (C₁-C₆)alkyle ; en particulier H ou Me, et
- R₅ à R₇ représentent, indépendamment les uns des autres, un atome d'hydrogène, un groupe (C₁-C₆)alkyle, de préférence méthyle, ou un groupe -C(O)NH-(C₁-C₆)alkyle, de préférence -C(O)NHMe ; en particulier H, Me ou -C(O)NHMe.

Le groupe R pourra être plus particulièrement un groupe choisi parmi :

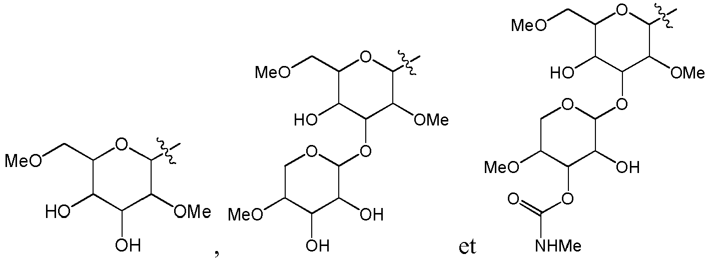

Compte tenu du nombre d'atomes de carbone asymétriques présents sur ces composés de formule (I), ces derniers peuvent prendre différentes configurations. Un composé selon l'invention pourra être en particulier un composé de stéréochimie définie par son mode de préparation, notamment susceptible d'être obtenu par un procédé comprenant les étapes successives suivantes :
(i) macération une ou plusieurs fois d'un lyophilisat d'une éponge de la famille *Verongidae* telle qu'une éponge *Suberea sp.* par exemple *Suberea creba,* avec une solution hydro-éthanolique (notamment eau/éthanol 10/90 v/v) ; suivie d'une filtration pour donner un filtrat ; puis concentration dudit filtrat pour donner un sirop aqueux,
(ii) ajout d'eau au sirop aqueux obtenu à l'étape (i) précédente, extraction une ou plusieurs fois avec de l'acétate d'éthyle et séparation des phases aqueuse et organique résultantes ; concentration de la phase organique ou des phases organiques réunies ainsi obtenu(e)s pour donner un extrait sec,
(iii) isolement à partir de l'extrait sec obtenu à l'étape (ii) précédente d'un composé de formule (I) avec R représentant : 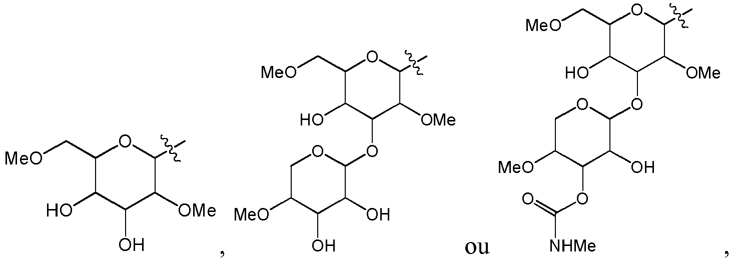
(iv) éventuellement hydrolyse du composé de formule (I) obtenu à l'étape précédente pour donner un composé de formule (II) suivante : 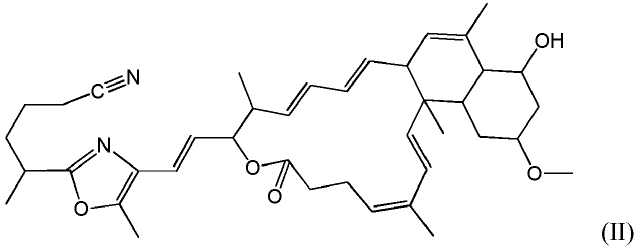 et substitution de la fonction OH du composé de formule (II) pour donner un composé de formule (I) tel que défini précédemment, et
(v) éventuellement salification, hydratation ou solvatation du composé de formule (I) obtenu à l'étape (iii) ou (iv) précédente pour donner un sel, hydrate ou solvate pharmaceutiquement acceptable de celui-ci.

Dans le cadre de ce procédé, l'éponge de la famille *Verongidae* telle qu'une éponge *Suberea sp.* par exemple *Suberea creba,* utilisée pourra être originaire de Nouvelle Calédonie.

Le composé de formule (I) pourra être choisi plus particulièrement parmi : 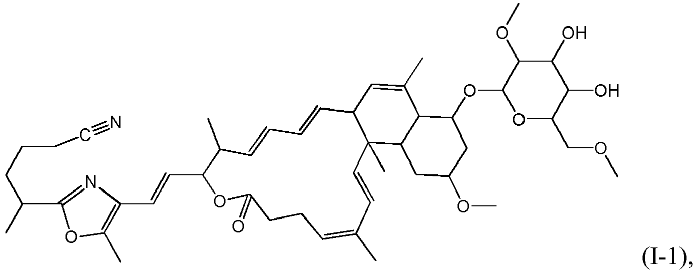 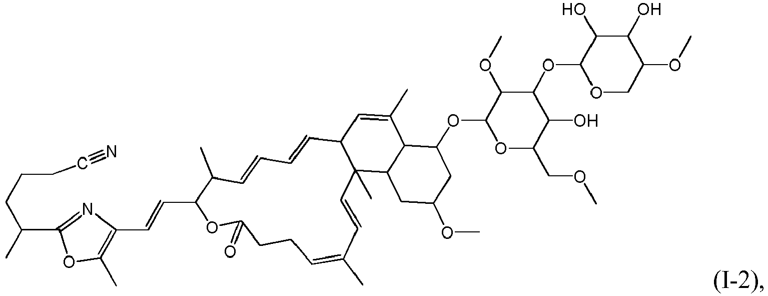 et

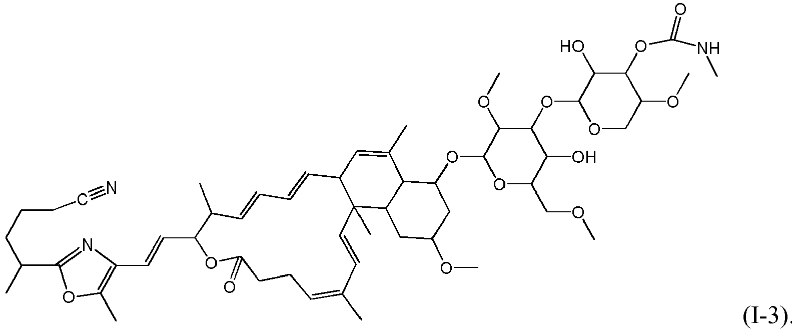

La présente invention a également pour objet un composé de formule (I) tel que défini ci-dessus pour son utilisation en tant que médicament, notamment destiné au traitement du cancer.

La présente invention concerne également l'utilisation d'un composé de formule (I) tel que décrit précédemment pour la préparation d'un médicament, destiné notamment au traitement du cancer.

L'invention concerne aussi une méthode de traitement du cancer comprenant l'administration à une personne en ayant besoin d'une dose efficace d'un composé de formule (I) tel que défini ci-dessus.

Le cancer peut être notamment les tumeurs solides ou non solides, tels que le mélanome, le cancer colorectal, le cancer du poumon, le cancer de la prostate, le cancer du foie, le cancer du sein, le cancer de l'utérus, le cancer de l'estomac, le cancer du pancréas, le cancer de la vessie, le cancer de l'ovaire, les cancers de la tête et du cou, le cancer du cerveau, la leucémie, les lymphomes (dont le lymphome de Burkitt) et les myélomes.

La présente invention a également pour objet une composition pharmaceutique comprenant au moins un composé de formule (I) tel que défini ci-dessus et un excipient pharmaceutiquement acceptable.

Les compositions pharmaceutiques de l'invention pourront être formulées par exemple pour une administration par voie intraveineuse ou orale.

Les composés de l'invention en tant que principes actifs peuvent être utilisés à des doses comprises entre 0,01 mg et 1000 mg par jour, par exemple en une seule dose.

Dans un mode de réalisation particulier de l'invention, la composition pharmaceutique est utilisée en tant que médicament, par exemple pour le traitement du cancer.

La présente invention a également pour objet un procédé de préparation d'un composé tel que défini précédemment, comprenant les étapes successives suivantes :
(i) macération une ou plusieurs fois d'un lyophilisat d'une éponge de la famille *Verongidae* telle qu'une éponge *Suberea sp.* par exemple *Suberea creba,* avec une solution hydro-éthanolique (notamment eau/éthanol 10/90 v/v) ; suivie d'une filtration pour donner un filtrat ; puis concentration dudit filtrat pour donner un sirop aqueux,
(ii) ajout d'eau au sirop aqueux obtenu à l'étape (i) précédente, extraction une ou plusieurs fois avec de l'acétate d'éthyle et séparation des phases aqueuse et organique résultantes ; concentration de la phase organique ou des phases organiques réunies ainsi obtenu(e)s pour donner un extrait sec,
(iii) isolement à partir de l'extrait sec obtenu à l'étape (ii) précédente d'un composé de formule (I) avec R représentant : 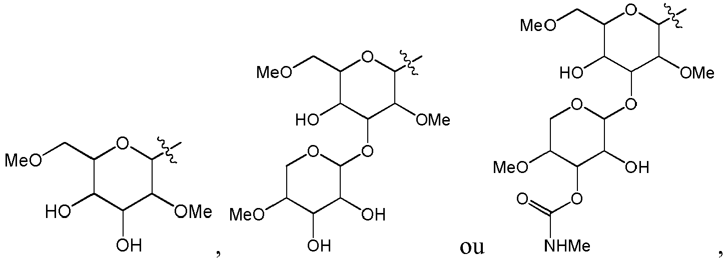
(iv) éventuellement hydrolyse du composé de formule (I) obtenu à l'étape précédente pour donner un composé de formule (II) suivante : 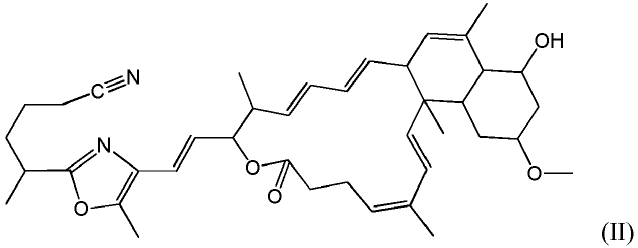 et substitution de la fonction OH du composé de formule (II) pour donner un composé de formule (I) tel que défini précédemment, et
(v) éventuellement salification, hydratation ou solvatation du composé de formule (I) obtenu à l'étape (iii) ou (iv) précédente pour donner un sel, hydrate ou solvate pharmaceutiquement acceptable de celui-ci.

Dans le cadre de ce procédé, l'éponge de la famille *Verongidae* telle qu'une éponge *Suberea sp.* par exemple *Suberea creba,* utilisée pourra être originaire de Nouvelle Calédonie.

L'étape de macération de l'étape (i) pourra être réalisée 1 à 5 fois, notamment 4 fois.

L'étape d'extraction de l'étape (ii) pourra être réalisée 1 à 5 fois, notamment 3 fois.

L'isolement du composé selon l'invention à l'étape (iii) pourra être réalisé notamment par une chromatographie sur gel de silice. Le produit obtenu pourra alors être purifié par des techniques bien connues de l'homme du métier, et notamment par chromatographie liquide haute performance (HPLC).

Les étapes d'hydrolyse et de substitution de l'étape (iv) et de salification, hydratation et solvatation de l'étape (v) pourront être réalisées par des techniques bien connues de l'homme du métier.

Le composé ainsi obtenu pourra être séparé du milieu réactionnel par des méthodes bien connues de l'homme du métier, comme par exemple par extraction, évaporation du solvant ou encore par précipitation et filtration.

Le composé pourra par ailleurs être purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

L'invention est plus particulièrement décrite de manière non limitative dans les exemples qui suivent.

### EXEMPLES

Dans les exemples ci-dessous, les abréviations suivantes ont été utilisées :
- ACN: Acétonitrile
- ESIMS: Spectrométrie de masse à ionisation par électrospray
- HPLC: Chromatographie liquide haute performance
- HRESITOFMS: Spectrométrie de masse haute résolution par temps de vol avec ionisation par électrospray
- RMN: Résonance magnétique nucléaire

### 1. Préparation des composés de formule (I)

Des échantillons de l'éponge marine *Suberea sp.* par exemple *Suberea creba,* ont été récoltés en Nouvelle Calédonie en 2000, puis lyophilisés pour donner un lyophilisat de 150 g. Ce lyophilisat a successivement été macéré quatre fois pendant 1 heure et sous agitation avec une solution hydro-alcoolique 10:90 (signifiant 10 % d'eau et 90 % d'éthanol en volume). Les filtrats obtenus (4 Litres au total) ont été regroupés, filtrés et concentrés à l'aide d'un évaporateur rotatif jusqu'à l'obtention d'un sirop aqueux. Ce dernier sirop aqueux a successivement été partitionné trois fois avec de l'acétate d'éthyle. Les phases organiques ont été regroupées et séchées jusqu'à l'obtention d'un extrait organique brut sec (2,55 g).

L'extrait organique a présenté une inhibition de croissance cellulaire de l'ordre de 45 % à la fois à une concentration de 10 et 1 µg/mL (résultats obtenus respectivement de 43,4 % et 46,7 %) sur la lignée cellulaire de mélanome métastatique humain WM266-4, montrant un effet cytostatique.

Une chromatographie type « Flash » (ou « par étapes ») sur colonne de silice normale a été réalisée sur l'extrait organique brut avec une élution en polarité croissante de cyclohexane et d'acétate d'éthyle puis d'acétate d'éthyle et de méthanol afin obtenir 13 fractions. L'ensemble des fractions ont été testées sur la lignée WM-266-4. Seules les fractions N° 6 et N° 8 ont présenté un pourcentage d'inhibition de 53 et 47 % respectivement à 10 µg/mL.

Les purifications finales ont été réalisées par HPLC préparative en phase inverse (RP C18) en utilisant un gradient d'eau et d'acétonitrile pour obtenir les trois molécules actives suivantes :

| | **Molécule (1-1)** | **Molécule (1-2)** | **Molécule (1-3)** |
|---|---|---|---|
| UVmax (Eau-ACN) | 235 nm (large) | 235 nm (large) | 235 nm (large) |
| Quantité | 2 mg | 3,1 mg | 4,5 mg |
| Rendement par rapport au lyophilisat de l'éponge | 0,0013 % | 0,0021 % | 0,0030 % |

### Molécule (I-1) :

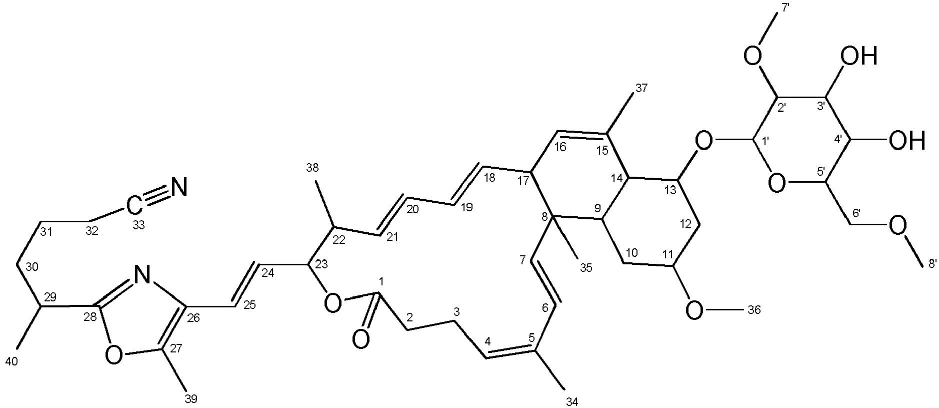

Formule brute : C₄₈H₆₈N₂O₁₀ - Masse exacte : 832,49
ESIMS m/z : 833,7 (M+H)⁺
RMN¹H (500 MHz, METHANOL-d₄) δ = 6.50 (1H, d, J = 16.4 Hz, H-6), 6.36 (1H, d, J = 15.4 Hz, H-25), 6.17 (1H, dd, J = 15.4 Hz, J = 8.2 Hz, H-24), 6.03 (1H, t, J = 10.4 Hz, H-20), 5.98 (1H, t, J = 10.4 Hz, H-19), 5.75 (1H, dd, J = 14.2 Hz, J = 9.8 Hz, H-18), 5.56 (1H, d, J = 16.4 Hz, H-7), 5.25 (2H, dd, J = 14.2 Hz, J = 9.8 Hz, H-21), 5.23 (1H, m, H-16), 5.17 (1H, dd, J = 9.0 Hz, J = 4.9 Hz, H-4), 5.11 (1H, dd, J = 10.1 Hz, J = 8.5 Hz, H-23), 3.67 (1H, dd, J = 10.7 Hz, J = 2.2 Hz, H-6'), 3.60 (3H, s, H-7'), 3.56 (1H, dd, J = 10.7 Hz, J = 5.0 Hz, H-6'), 3.48 (1H, m, H-4'), 3.48 (1H, m, H-2"), 3.42 (1H, t, J = 9.3 Hz, H-3'), 3.38 (1H, m, H-11), 3.35 (3H, s, H-8'), 3.33 (1H, m, H-5'), 3.31 (3H, s, H-36), 3.09 (1H, dd, J = 9.1 Hz, J = 7.9 Hz, H-2'), 2.92 (1H, m, H-29), 2.80 (1H, m, H-17), 2.67 (1H, s, H-14), 2.45 (1H, m, H-3), 2.33 (2H, td, J = 7.3 Hz, J = 3.8 Hz, H-32), 2.28 (1H, m, H-2), 2.28 (1H, m, H-22), 2.27 (3H, s, H-39), 2.20 (1H, d, J = 12.9 Hz, H-12), 2.14 (1H, m, H-3), 2.08 (1H, td, J = 13.6 Hz, J = 3.2 Hz, H-2), 1.87 (1H, m, H-9), 1.87 (1H, m, H-10), 1.87 (1H, m, H-30), 1.75 (3H, s, H-34), 1.72 (1H, m, H-30), 1.64 (2H, m, H-31), 1.63 (3H, s, H-37), 1.33 (1H, t, J = 12.0 Hz, H-10), 1.30 (3H, d, J = 6.9 Hz, H-40), 1.06 (1H, m, H-12), 0.98 (3H, d, J = 6.6 Hz, H-38).
RMN ¹³C (126 MHz, CDCl₃) δ = 172.6 (C-1), 165.3 (C-28), 145.1 (C-27), 139.1 (C-7), 136.3 (C-18), 133.8 (C-21), 133.3 (C-5), 132.4 (C-20), 132.0 (C-26), 129.8 (C-19), 128.4 (C-15), 127.5 (C-16), 127.1 (C-6), 126.4 (C-4), 126.3 (C-24), 122.5 (C-25), 119.4 (C-33), 104.7 (C-1') 82 (C-2'), 77.5 (C-23), 77.1 (C-13), 75.5 (C-11), 74.8 (C-5'), 71.7 (C-6'), 68.9 (C-4'), 61.1 (C-7'), 59.4 (C-8'), 55.8(C-36), 48.5 (C-17), 44.0 (C-22), 40.8 (C-8), 40.7 (C-9), 40.3 (C-14), 35.2 (C-2), 33.8 (C-30), 33.2 (C-29), 32.6 (C-12), 32.0 (C-10), 30.9 (C-35), 24.1 (C-3), 23.2 (C-31), 20.8 (C-37), 20.2 (C-34), 18.6 (C-40), 17.1 (C-32).

### Molécule (I-2):

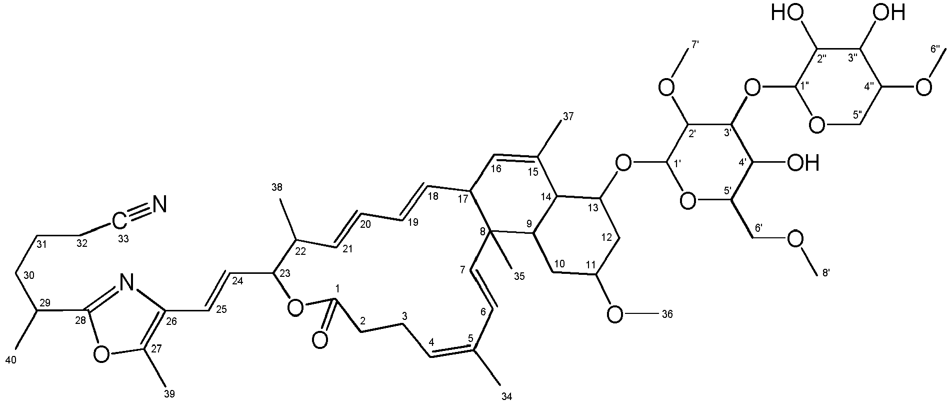

Formule brute : C₅₄H₇₈N₂O₁₄ - Masse exacte : 978,55
ESIMS m/z : 979,7 (M+H)⁺
HRESITOFMS m/z: 1001,53519 (M+Na)⁺, calculée pour C₅₄H₇₈N₂O₁₄Na₁ m/z 1001,53453
RMN¹H (500 MHz, METHANOL-d₄) δ = 6.50 (1H, d, J = 16.4 Hz, H-6), 6.36 (1H, d, J = 15.4 Hz, H-25), 6.17 (1H, dd, J = 15.4 Hz, J = 8.2 Hz, H-24), 6.03 (1H, t, J = 10.4 Hz, H-20), 5.98 (1H, t, J = 10.4 Hz, H-19), 5.75 (1H, dd, J = 14.2 Hz, J = 9.8 Hz, H-18), 5.56 (1H, d, J = 16.4 Hz, H-7), 5.25 (2H, dd, J = 14.2 Hz, J = 9.8 Hz, H-21), 5.23 (1H, m, H-16), 5.17 (1H, dd, J = 9.0 Hz, J = 4.9 Hz, H-4), 5.11 (1H, dd, J = 10.1 Hz, J = 8.5 Hz, H-23), 4.82 (1H, s, H-8"), 4.81 (1H, t, J = 8.8 Hz, H-3"), 4.44 (1H, d, J = 7.6 Hz, H-1"), 4.40 (1H, q, J = 2.8 Hz, H-13), 4.36 (1H, d, J = 7.9 Hz, H-1'), 4.08 (1H, dd, J = 11.2 Hz, J = 4.6 Hz, H-5"), 3.67 (1H, dd, J = 10.7 Hz, J = 2.2 Hz, H-6'), 3.60 (3H, s, H-7'), 3.56 (1H, dd, J = 10.7 Hz, J = 5.0 Hz, H-6'), 3.48 (1H, m, H-4'), 3.48 (1H, m, H-2"), 3.42 (1H, t, J = 9.3 Hz, H-3'), 3.38 (1H, m, H-11), 3.38 (1H, m, H-4"), 3.35 (3H, s, H-8'), 3.33 (1H, m, H-5'), 3.33 (1H, m, H-5"), 3.31 (3H, s, H-36), 3.09 (1H, dd, J = 9.1 Hz, J = 7.9 Hz, H-2'), 2.92 (1H, m, H-29), 2.80 (1H, m, H-17), 2.67 (1H, s, H-14), 2.45 (1H, m, H-3), 2.33 (2H, td, J = 7.3 Hz, J = 3.8 Hz, H-32), 2.28 (1H, m, H-2), 2.28 (1H, m, H-22), 2.27 (3H, s, H-39), 2.20 (1H, d, J = 12.9 Hz, H-12), 2.14 (1H, m, H-3), 2.08 (1H, td, J = 13.6 Hz, J = 3.2 Hz, H-2), 1.87 (1H, m, H-9), 1.87 (1H, m, H-10), 1.87 (1H, m, H-30), 1.75 (3H, s, H-34), 1.72 (1H, m, H-30), 1.64 (2H, m, H-31), 1.63 (3H, s, H-37), 1.33 (1H, t, J = 12.0 Hz, H-10), 1.30 (3H, d, J = 6.9 Hz, H-40), 1.06 (1H, m, H-12), 0.98 (3H, d, J = 6.6 Hz, H-38).
RMN¹³C (126 MHz, CDCl₃) δ = 172.6 (C-1), 165.3 (C-28), 145.1 (C-27), 139.1 (C-7), 136.3 (C-18), 133.8 (C-21), 133.3 (C-5), 132.4 (C-20), 132.0 (C-26), 129.8 (C-19), 128.4 (C-15), 127.5 (C-16), 127.1 (C-6), 126.4 (C-4), 126.3 (C-24), 122.5 (C-25), 119.4 (C-33), 104.7 (C-1') 102.6 (C-1"), 86.9 (C-3"), 82 (C-2'), 77.5 (C-23), 77.1 (C-13), 76.7 (C-3"), 76.6 (C-4"), 75.5 (C-11), 74.8 (C-5'), 72.5 (C-2"), 71.7 (C-6'), 68.9 (C-4'), 63.3 (C-5"), 61.1 (C-7'), 59.4 (C-8'), 58.8 (C-6"), 55.8(C-36), 48.5 (C-17), 44.0 (C-22), 40.8 (C-8), 40.7 (C-9), 40.3 (C-14), 35.2 (C-2), 33.8 (C-30), 33.2 (C-29), 32.6 (C-12), 32.0 (C-10), 30.9 (C-35), 24.1 (C-3), 23.2 (C-31), 20.8 (C-37), 20.2 (C-34), 18.6 (C-40), 17.1 (C-32).

### Molécule (I-3):

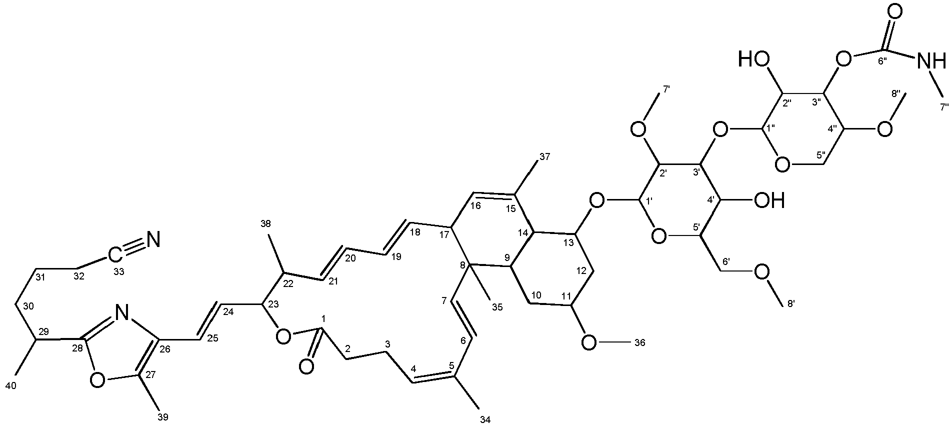

Formule brute : C₅₆H₈₁N₃O₁₅ - Masse exacte : 1035,57
ESIMS m/z : 1038,8 (M+H)⁺
RMN¹H (500 MHz, METHANOL-d₄) δ = 6.50 (1H, d, J = 16.4 Hz, H-6), 6.36 (1H, d, J = 15.4 Hz, H-25), 6.17 (1H, dd, J = 15.4 Hz, J = 8.2 Hz, H-24), 6.03 (1H, t, J = 10.4 Hz, H-20), 5.98 (1H, t, J = 10.4 Hz, H-19), 5.75 (1H, dd, J = 14.2 Hz, J = 9.8 Hz, H-18), 5.56 (1H, d, J = 16.4 Hz, H-7), 5.25 (2H, dd, J = 14.2 Hz, J = 9.8 Hz, H-21), 5.23 (1H, m, H-16), 5.17 (1H, dd, J = 9.0 Hz, J = 4.9 Hz, H-4), 5.11 (1H, dd, J = 10.1 Hz, J = 8.5 Hz, H-23), 4.82 (1H, s, H-8"), 4.81 (1H, t, J = 8.8 Hz, H-3"), 4.44 (1H, d, J = 7.6 Hz, H-1"), 4.40 (1H, q, J = 2.8 Hz, H-13), 4.36 (1H, d, J = 7.9 Hz, H-1'), 4.08 (1H, dd, J = 11.2 Hz, J = 4.6 Hz, H-5"), 3.67 (1H, dd, J = 10.7 Hz, J = 2.2 Hz, H-6'), 3.60 (3H, s, H-7'), 3.56 (1H, dd, J = 10.7 Hz, J = 5.0 Hz, H-6'), 3.48 (1H, m, H-4'), 3.48 (1H, m, H-2"), 3.42 (1H, t, J = 9.3 Hz, H-3'), 3.41 (3H, s, H-6"), 3.38 (1H, m, H-11), 3.38 (1H, m, H-4"), 3.35 (3H, s, H-8'), 3.33 (1H, m, H-5'), 3.33 (1H, m, H-5"), 3.31 (3H, s, H-36), 3.09 (1H, dd, J = 9.1 Hz, J = 7.9 Hz, H-2'), 2.92 (1H, m, H-29), 2.82 (3H, d, J = 5.0 Hz, H-9"), 2.80 (1H, m, H-17), 2.67 (1H, s, H-14), 2.45 (1H, m, H-3), 2.33 (2H, td, J = 7.3 Hz, J = 3.8 Hz, H-32), 2.28 (1H, m, H-2), 2.28 (1H, m, H-22), 2.27 (3H, s, H-39), 2.20 (1H, d, J = 12.9 Hz, H-12), 2.14 (1H, m, H-3), 2.08 (1H, td, J = 13.6 Hz, J = 3.2 Hz, H-2), 1.87 (1H, m, H-9), 1.87 (1H, m, H-10), 1.87 (1H, m, H-30), 1.75 (3H, s, H-34), 1.72 (1H, m, H-30), 1.64 (2H, m, H-31), 1.63 (3H, s, H-37), 1.33 (1H, t, J = 12.0 Hz, H-10), 1.30 (3H, d, J = 6.9 Hz, H-40), 1.06 (1H, m, H-12), 0.98 (3H, d, J = 6.6 Hz, H-38).
RMN¹³C (126 MHz, CDCl₃) δ = 172.6 (C-1), 165.3 (C-28), 157.1 (C-6"), 145.1 (C-27), 139.1 (C-7), 136.3 (C-18), 133.8 (C-21), 133.3 (C-5), 132.4 (C-20), 132.0 (C-26), 129.8 (C-19), 128.4 (C-15), 127.5 (C-16), 127.1 (C-6), 126.4 (C-4), 126.3 (C-24), 122.5 (C-25), 119.4 (C-33), 104.7 (C-1') 102.6 (C-1"), 86.9 (C-3"), 82 (C-2'), 77.5 (C-23), 77.1 (C-13), 76.7 (C-3"), 76.6 (C-4"), 75.5 (C-11), 74.8 (C-5'), 72.5 (C-2"), 71.7 (C-6'), 68.9 (C-4'), 63.3 (C-5"), 61.1 (C-7'), 59.4 (C-8'), 58.8 (C-8"), 55.8(C-36), 48.5 (C-17), 44.0 (C-22), 40.8 (C-8), 40.7 (C-9), 40.3 (C-14), 35.2 (C-2), 33.8 (C-30), 33.2 (C-29), 32.6 (C-12), 32.0 (C-10), 30.9 (C-35), 27.7 (C-7"), 24.1 (C-3), 23.2 (C-31), 20.8 (C-37), 20.2 (C-34), 18.6 (C-40), 17.1 (C-32).

### 2. Activité biologique des composés de formule (I)

Les molécules (I-1), (I-2) et (I-3) ont été mises en évidence au cours d'un test anti-prolifératif sur une lignée cellulaire de mélanome métastatique WM266-4. Elles se sont avérées très cytotoxiques sur un ensemble de sept autres lignées tumorales (A549 : lignée cellulaire issue d'un adénocarcinome pulmonaire humain ; BxPC3 : lignée cellulaire issue d'un adénocarcinome pancréatique humain ; KB : lignée cellulaire issue d'un carcinome oral humain ; KB-V1 : lignée cellulaire issue d'un carcinome du col de l'utérus humain résistant à la vinblastine ; LoVo : lignée cellulaire issue d'un adénocarcinome colique humain ; Namalwa : lignée cellulaire issue d'un lymphome de Burkitt ; et SkoV3 : lignée cellulaire issue d'un adénocarcinome ovarien humain).

Les cellules sont ensemencées en plaque 96 puits dans du milieu RPMI 1640 sans rouge de phénol (Seromed) auquel est ajouté 10 % de sérum de veau foetal (100 µL/puits, 1 à 3 10⁴cellules/mL selon la lignée considérée). Après une incubation de 24 heures à 37°C dans un incubateur à 5 % CO₂, le milieu est remplacé par le même milieu contenant en outre le composé à tester (diverses concentrations étant utilisées) puis les plaques sont incubées 48 heures supplémentaires. La survie cellulaire est évaluée par mesure de la luminescence après relargage de l'ATP dans le milieu en utilisant les solutions de lyse cellulaire, de luciférase et de luciférine contenus dans le kit ATP-lite-M^{™} comme cela est recommandé par le fabricant (Packard, Rungis, France). Chaque condition expérimentale a été testée au moins trois fois en sextuplet.

Les résultats obtenus sont présentés dans le tableau suivant.

| **Molécule** | **CE50 pour diverses lignées cellulaires tumorales (en M)** | | | | | | |
|---|---|---|---|---|---|---|---|
| | **A549** | **BxPC3** | **KB** | **KB-V1** | **LoVo** | **Namalwa** | **SkoV3** |
| **(I-1)** | 1,1.10⁻¹¹ | 2,2.10⁻¹⁰ | 3,1.10⁻¹⁰ | 7,0.10⁻¹⁰ | 4,0.10⁻¹¹ | 3,1.10⁻¹⁰ | 3,6.10⁻⁵ |
| **(1-2)** | <10⁻¹² | 2,1.10⁻¹⁰ | 3,0.10⁻¹⁰ | 4,9.10⁻¹⁰ | 1,1.10⁻¹¹ | 3,6.10⁻¹⁰ | 1,3.10⁻⁶ |
| **(1-3)** | <10⁻¹² | 1,2.10⁻¹¹ | 2,4.10⁻¹¹ | 7,3.10⁻¹⁰ | <10⁻¹¹ | 3,2.10⁻¹⁰ | 3,2.10⁻⁷ |

## Revendications

1. Composé de formule (I) suivante : 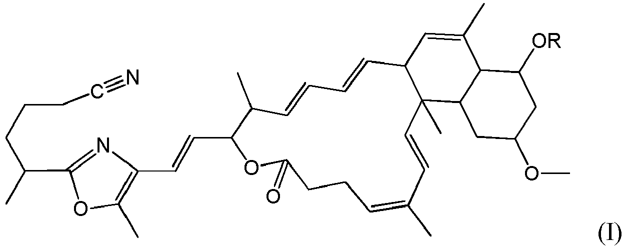 ou un sel, hydrate ou solvate pharmaceutiquement acceptable de celui-ci,
dans laquelle R représente un résidu de monosaccharide ou disaccharide, les fonctions hydroxyles du résidu de monosaccharide ou disaccharide étant, indépendamment les unes des autres, éventuellement substituées par un groupe (C₁-C₆)alkyle, de préférence méthyle, ou -C(O)NH-(C₁-C₆)alkyle, de préférence -C(O)NHMe.

2. Composé selon la revendication 1, **caractérisé en ce que** R est le groupe suivant : 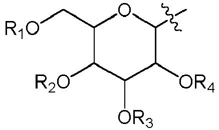 dans lequel les groupes R₁ à R₄ représentent, indépendamment les uns des autres, un atome d'hydrogène, un groupe (C₁-C₆)alkyle, de préférence méthyle, un groupe -C(O)NH-(C₁-C₆)alkyle, de préférence -C(O)NHMe, ou un résidu de monosaccharide de formule suivante : 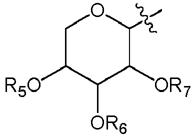 dans lequel les groupes R₅ à R₇ représentent, indépendamment les uns des autres, un atome d'hydrogène, un groupe (C₁-C₆)alkyle, de préférence méthyle, ou un groupe - C(O)NH-(C₁-C₆)alkyle, de préférence -C(O)NHMe,
un seul groupe R₁ à R₄ pouvant représenter un résidu de monosaccharide.

3. Composé selon la revendication 2, **caractérisé en ce que** R₁ = R₄ = Me, R₂ = H et R₃ = H ou un résidu de monosaccharide de formule suivante : 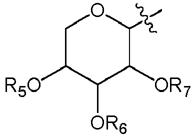 avec R₅ à R₇ tels que définis à la revendication 2.

4. Composé selon la revendication 2, **caractérisé en ce que** R représente un groupe : 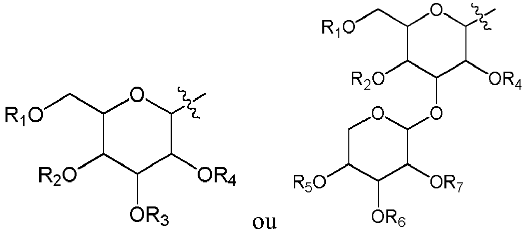 dans lequel :
- R₁ à R₄ représentent, indépendamment les uns des autres, un atome d'hydrogène, un groupe (C₁-C₆)alkyle, de préférence méthyle, ou un groupe -C(O)NH-(C₁-C₆)alkyle, de préférence -C(O)NHMe, et
- R₅ à R₇ représentent, indépendamment les uns des autres, un atome d'hydrogène, un groupe (C₁-C₆)alkyle, de préférence méthyle, ou un groupe -C(O)NH-(C₁-C₆)alkyle, de préférence -C(O)NHMe.

5. Composé selon la revendication 4, **caractérisé en ce que** R représente un groupe choisi parmi :

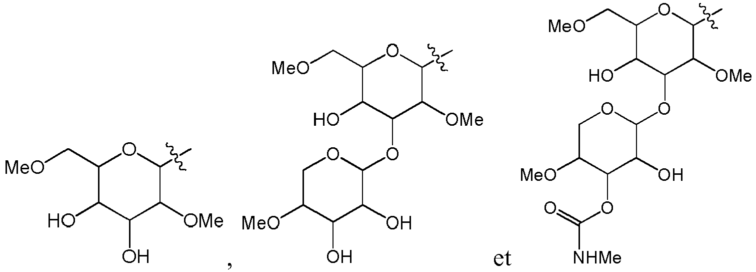

6. Composé selon l'une quelconque des revendications 1 à 5, **caractérisé en ce qu'**il est susceptible d'être obtenu par un procédé comprenant les étapes successives suivantes :
(i) macération une ou plusieurs fois d'un lyophilisat d'une éponge de la famille *Verongidae* telle qu'une éponge *Suberea sp.* par exemple *Suberea creba,* avec une solution hydro-éthanolique (notamment eau/éthanol 10/90 v/v) ; suivie d'une filtration pour donner un filtrat ; puis concentration dudit filtrat pour donner un sirop aqueux,
(ii) ajout d'eau au sirop aqueux obtenu à l'étape (i) précédente, extraction une ou plusieurs fois avec de l'acétate d'éthyle et séparation des phases aqueuse et organique résultantes ; concentration de la phase organique ou des phases organiques réunies ainsi obtenu(e)s pour donner un extrait sec,
(iii) isolement à partir de l'extrait sec obtenu à l'étape (ii) précédente d'un composé de formule (I) selon la revendication 1 avec R représentant : 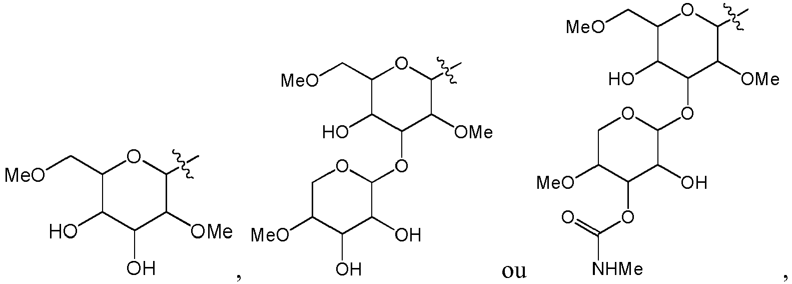
(iv) éventuellement hydrolyse du composé de formule (I) obtenu à l'étape précédente pour donner un composé de formule (II) suivante : 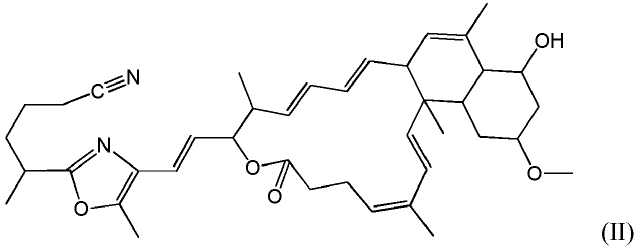 et substitution de la fonction OH du composé de formule (II) pour donner un composé de formule (I) tel que défini à la revendication 1, et
(v) éventuellement salification, hydratation ou solvatation du composé de formule (I) obtenu à l'étape (iii) ou (iv) précédente pour donner un sel, hydrate ou solvate pharmaceutiquement acceptable de celui-ci.

7. Composé selon l'une quelconque des revendications 1 à 6, **caractérisé en ce qu'**il est choisi parmi : 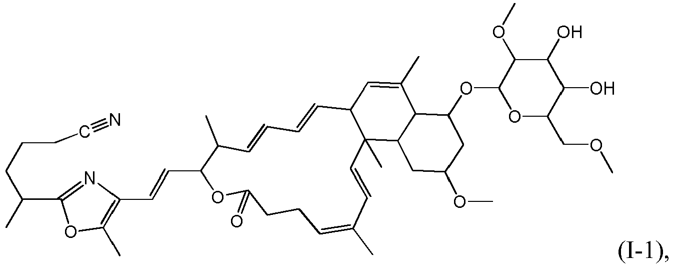 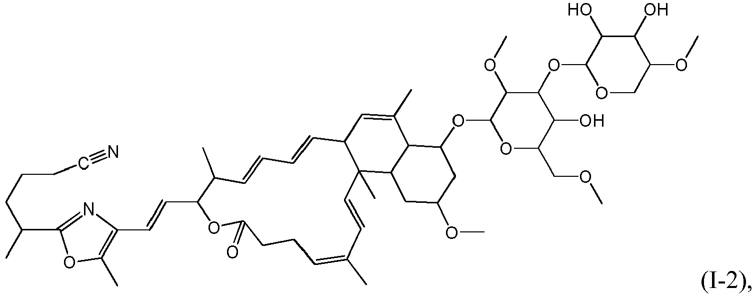 et

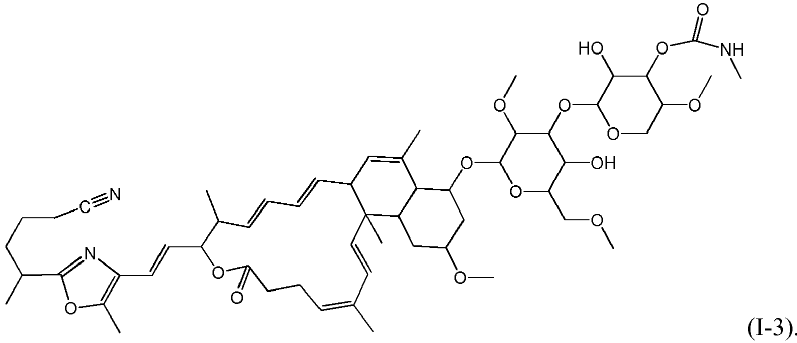

8. Composé selon l'une quelconque des revendications 1 à 7 pour son utilisation en tant que médicament.

9. Composé selon l'une quelconque des revendications 1 à 7 pour son utilisation dans le traitement du cancer.

10. Composition pharmaceutique comprenant au moins un composé de formule (I) selon l'une quelconque des revendications 1 à 7 et un excipient pharmaceutiquement acceptable.

11. Composition pharmaceutique selon la revendication 10 pour son utilisation dans le traitement du cancer.

12. Procédé de préparation d'un composé selon l'une quelconque des revendications 1 à 7, comprenant les étapes successives suivantes :
(i) macération une ou plusieurs fois d'un lyophilisat d'une éponge de la famille *Verongidae* telle qu'une éponge *Suberea sp.* par exemple *Suberea creba,* avec une solution hydro-éthanolique (notamment eau/éthanol 10/90, v/v) ; suivie d'une filtration pour donner un filtrat ; puis concentration dudit filtrat pour donner un sirop aqueux,
(ii) ajout d'eau au sirop aqueux obtenu à l'étape (i) précédente, extraction une ou plusieurs fois avec de l'acétate d'éthyle et séparation des phases aqueuse et organique résultantes ; concentration de la phase organique ou des phases organiques réunies ainsi obtenu(e)s pour donner un extrait sec,
(iii) isolement à partir de l'extrait sec obtenu à l'étape (ii) précédente d'un composé de formule (I) selon la revendication 1 avec R représentant : 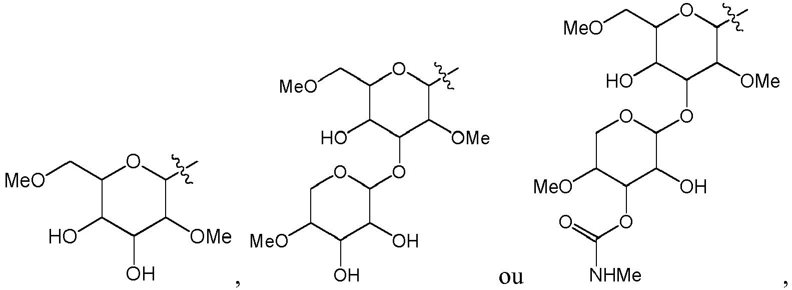
(iv) éventuellement hydrolyse du composé de formule (I) obtenu à l'étape précédente pour donner un composé de formule (II) suivante : 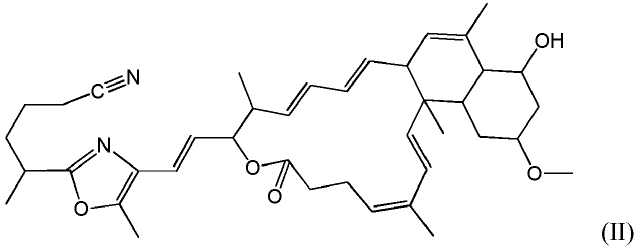 et substitution de la fonction OH du composé de formule (II) pour donner un composé de formule (I) selon la revendication 1, et
(v) éventuellement salification, hydratation ou solvatation du composé de formule (I) obtenu à l'étape (iii) ou (iv) précédente pour donner un sel, hydrate ou solvate pharmaceutiquement acceptable de celui-ci.

## Patentansprüche

1. Verbindung der folgenden Formel (I): 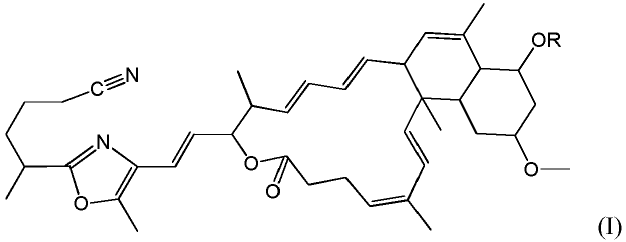 oder ein pharmazeutisch akzeptables Salz, Hydrat oder Solvat davon,
worin R ein Mono- oder Disaccharidrest bedeutet, und die Hydroxylfunktionen des Mono- oder Disaccharidrestes unabhängig voneinander gegebenenfalls durch eine (C₁-C₆)-Alkylgruppe, bevorzugt Methyl oder -C(O)NH-(C₁-C₆)-Alkylgruppe, bevorzugt -C(O)NHMe substituiert sind.

2. Verbindung nach Anspruch 1, **dadurch gekennzeichnet, dass** R die folgende Gruppe ist: 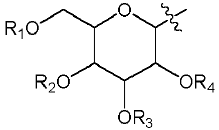 in der die Gruppen R₁ bis R₄ unabhängig voneinander ein Wasserstoffatom, eine (C₁-C₆)-Alkylgruppe, bevorzugt Methyl, eine -C(O)NH-(C₁-C₆)-Alkylgruppe, bevorzugt -C(O)NHMe, oder ein Monosaccharidrest mit folgender Formel sind: 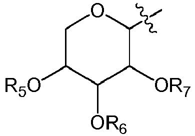 in der die Gruppen R₅ bis R₇ unabhängig voneinander ein Wasserstoffatom, eine (C₁-C₆)-Alkylgruppe, bevorzugt Methyl, oder eine C(O)NH-(C₁-C₆)-Alkylgruppe, bevorzugt -C(O)NHMe, sind:
wobei eine einzige Gruppe R₁ bis R₄ ein Monosaccharidrest sein kann.

3. Verbindung nach Anspruch 2, **dadurch gekennzeichnet, dass** R₁ = R₄ = Me, R₂ = H und R₃ = H oder ein Monosaccharidrest mit folgender Formel ist: 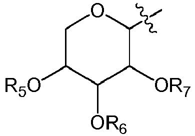 mit R₅ bis R₇ wie im Anspruch 2 definiert.

4. Verbindung nach Anspruch 2, **dadurch gekennzeichnet, dass** R eine Gruppe ist: 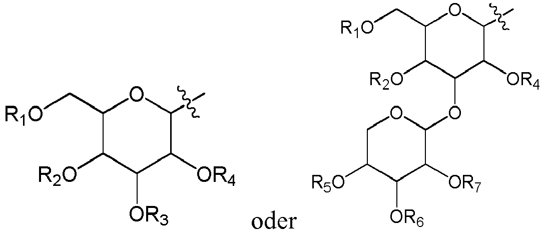 in der:
- R₁ bis R₄ unabhängig voneinander ein Wasserstoffatom, eine (C₁-C₆)-Alkylgruppe, bevorzugt Methyl, oder eine -C(O)NH-(C₁-C₆)Alkylgruppe, bevorzugt -C(O)NHMe sind, und
- R₅ bis R₇ unabhängig voneinander ein Wasserstoffatom, eine (C₁-C₆)-Alkylgruppe, bevorzugt Methyl, oder eine C(O)NH-(C₁-C₆)Alkylgruppe, bevorzugt -C(O)NHMe sind.

5. Verbindung nach Anspruch 4, **dadurch gekennzeichnet, dass** R eine Gruppe ist, die gewählt wird unter:

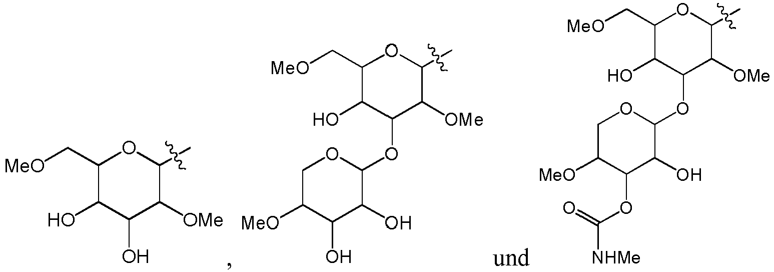

6. Verbindung nach einem beliebigen der Ansprüche 1 bis 5, **dadurch gekennzeichnet, dass** sie durch ein Verfahren erzielt werden kann, das die folgenden Schritte nacheinander umfasst:
(i) ein- oder mehrmalige Mazeration eines Lyophilisats eines Schwamms der Familie *Verongidae* wie eines *Suberea sp.* Schwamms zum Beispiel *Suberea creba,* in einer hydro-ethanolischen Lösung (insbesondere Wasser/Ethanol 10/90 v/v); gefolgt von einer Filtration, um zu einem Filtrat zu gelangen; dann Konzentration des besagten Filtrats, so dass ein wässriger Sirup entsteht,
(ii) Hinzufügung von Wasser zum wässrigen Sirup, der im vorherigen Schritt (i) erzielt wurde, ein- oder mehrmalige Extraktion mit Ethylacetat und Trennung der resultierenden wässrigen und organischen Phasen; Konzentration der somit erzielten organischen Phase oder der vereinten organischen Phasen, um zu einem Trockenextrakt zu gelangen,
(iii) aus dem im vorherigen Schritt (ii) erzielten Trockenextrakt Isolieren einer Verbindung mit der Formel (I) nach Anspruch 1, wobei R: 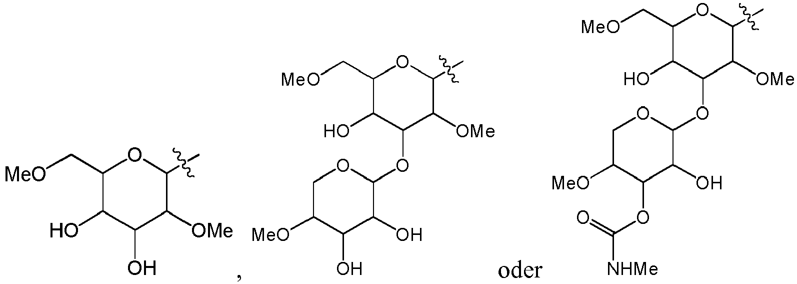 ist,
(iv) eventuell Hydrolyse der im vorherigen Schritt erzielten Verbindung mit der Formel (I), um zu einer Verbindung mit folgender Formel (II) zu gelangen: 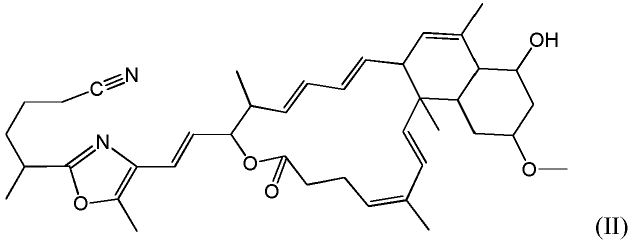 und Substitution der OH Funktion der Verbindung mit der Formel (II), um zu einer Verbindung mit der Formel (I) zu gelangen, wie im Anspruch 1 definiert ist, und
(v) eventuell Salzbildung, Hydrieren oder Solvatation der im vorherigen Schritt (iii) oder (iv) erzielten Verbindung mit der Formel (I), um zu einem pharmazeutisch akzeptablen Salz, Hydrat oder Solvat davon zu gelangen.

7. Verbindung nach einem beliebigen der Ansprüche 1 bis 6, **dadurch gekennzeichnet, dass** sie gewählt wird unter: 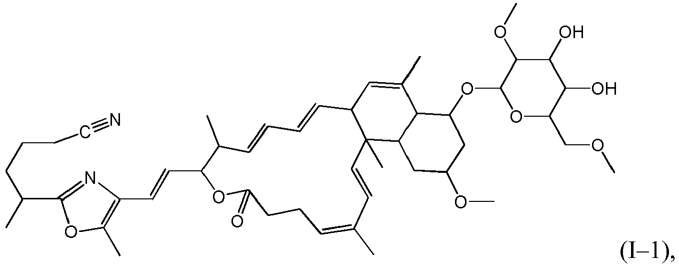 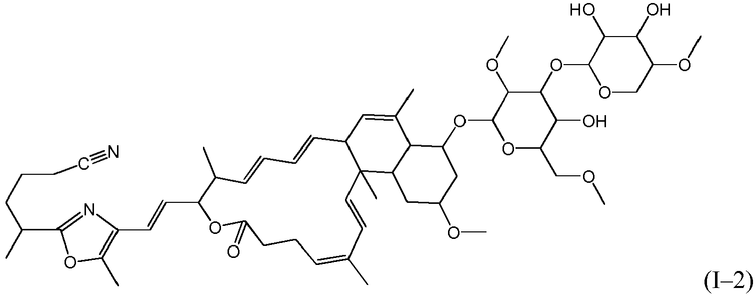 und

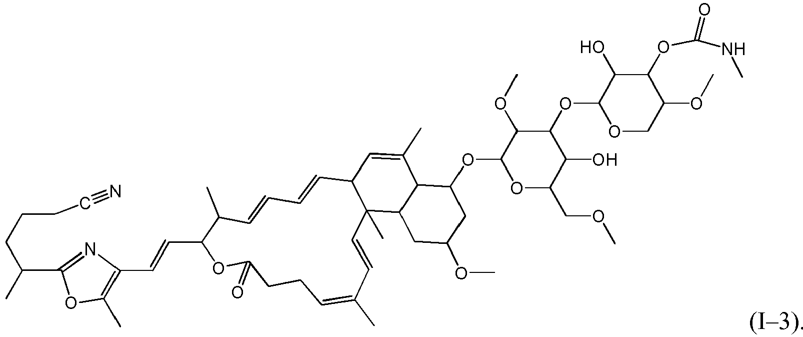

8. Verbindung nach einem beliebigen der Ansprüche 1 bis 7 für ihre Verwendung als Medikament.

9. Verbindung nach einem beliebigen der Ansprüche 1 bis 7 für ihre Verwendung in der Krebsbehandlung.

10. Pharmazeutische Verbindung, die zumindest eine Verbindung mit der Formel (I) nach einem beliebigen der Ansprüche 1 bis 7 und einen pharmazeutisch akzeptablen Wirkstoff umfasst.

11. Pharmazeutische Verbindung nach Anspruch 10 für ihre Verwendung in der Krebsbehandlung.

12. Verfahren zur Herstellung einer Verbindung nach einem beliebigen der Ansprüche 1 bis 7, das die folgenden Schritte nacheinander umfasst:
(i) ein- oder mehrmalige Mazeration eines Lyophilisats eines Schwamms der Familie *Verongidae* wie eines *Suberea sp.* Schwamms zum Beispiel *Suberea creba,* in einer hydro-ethanolischen Lösung (insbesondere Wasser/Ethanol 10/90 v/v); gefolgt von einer Filtration, um zu einem Filtrat zu gelangen; dann Konzentration des besagten Filtrats, so dass ein wässriger Sirup entsteht,
(ii) Hinzufügung von Wasser zum wässrigen Sirup, der im vorherigen Schritt (i) erzielt wurde, ein- oder mehrmalige Extraktion mit Ethylacetat und Trennung der resultierenden wässrigen und organischen Phasen; Konzentration der somit erzielten organischen Phase oder der vereinten organischen Phasen, um zu einem Trockenextrakt zu gelangen,
(iii) aus dem im vorherigen Schritt (ii) erzielten Trockenextrakt Isolieren einer Verbindung mit der Formel (I) nach Anspruch 1, wobei R: 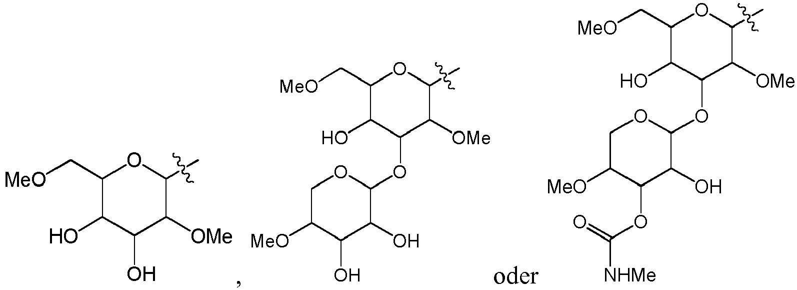 ist,
(iv) eventuell Hydrolyse der im vorherigen Schritt erzielten Verbindung mit der Formel (I), um zu einer Verbindung mit folgender Formel (II) zu gelangen: 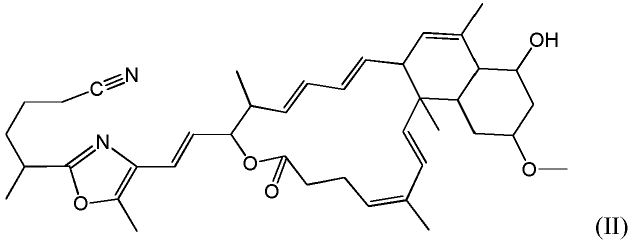 und Substitution der OH Funktion der Verbindung mit der Formel (II), um zu einer Verbindung mit der Formel (I) zu gelangen, wie im Anspruch 1 definiert ist, und
(v) eventuell Salzbildung, Hydrieren oder Solvatation der im vorherigen Schritt (iii) oder (iv) erzielten Verbindung mit der Formel (I), um zu einem pharmazeutisch akzeptablen Salz, Hydrat oder Solvat davon zu gelangen.

## Claims

1. A compound of the following formula (I): 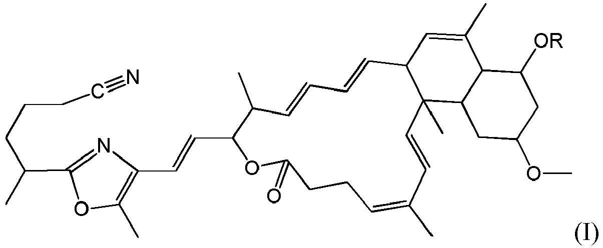 or a pharmaceutically acceptable salt, hydrate or solvate thereof,
in which R is a monosaccharide or disaccharide residue, the hydroxyl functional groups of the monosaccharide or disaccharide residue are, independently of each other, optionally substituted with a (C₁-C₆)alkyl group, preferably a methyl group, or a -C(O)NH-(C₁-C₆)alkyl group, preferably a -C(O)NHMe group.

2. The compound according to claim 1, **characterized in that** R is the following group: 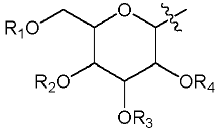 in which the R₁ to R₄ groups are, independently of each other, a hydrogen atom, a (C₁-C₆)alkyl group, preferably a methyl group, a -C(O)NH-(C₁-C₆)alkyl group, preferably a -C(O)NHMe group, or a monosaccharide residue of the following formula: 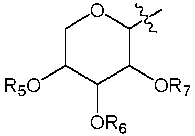 in which the R₅ to R₇ groups are, independently of each other, a hydrogen atom, a (C₁-C₆)alkyl group, preferably a methyl group, or a -C(O)NH-(C₁-C₆)alkyl group, preferably a -C(O)NHMe group,
wherein only one R₁ to R₄ group may be a monosaccharide residue.

3. The compound according to claim 2, **characterized in that** R₁ = R₄ = Me, R₂ = H and R₃ = H or a monosaccharide residue of the following formula: 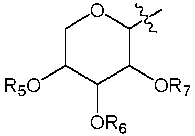 with R₅ with R₇ as defined in claim 2.

4. The compound according to claim 2, **characterized in that** R is a: 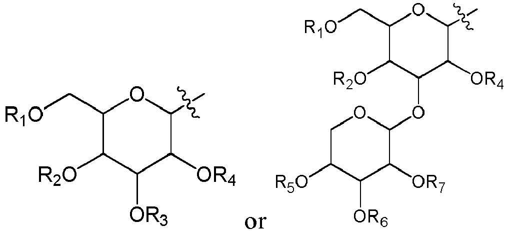 group
in which:
- R₁ to R₄ are, independently of each other, a hydrogen atom, a (C₁-C₆)alkyl group, preferably a methyl group, or a -C(O)NH-(C₁-C₆)alkyl group, preferably a -C(O)NHMe group, and
- R₅ to R₇ are, independently of each other, a hydrogen atom, a (C₁-C₆)alkyl group, preferably a methyl group, or a -C(O)NH-(C₁-C₆)alkyl group, preferably a -C(O)NHMe group.

5. The compound according to claim 4, **characterized in that** R is a group selected from:

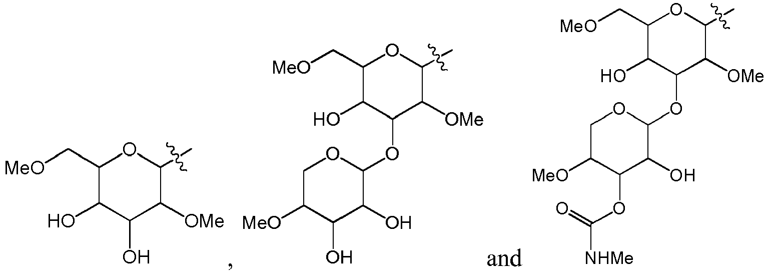

6. The compound according to any one of claims 1 to 5, **characterized in that** it is obtainable by a method comprising the following successive steps:
(i) one or more macerations of a lyophilizate of a sponge of the family *Verongidae,* such as a *Suberea sp.* sponge, for example *Suberea creba,* with a water/ethanol solution (in particular 10/90 water/ethanol, v/v); followed by filtration to yield a filtrate; then concentration of said filtrate to yield an aqueous syrup,
(ii) addition of water to the aqueous syrup obtained in the preceding step (i), one or more extractions with ethyl acetate and separation of the resulting aqueous and organic phases; concentration of the organic phase or the combined organic phases thus obtained to yield a dry extract,
(iii) isolation from the dry extract obtained in the preceding step (ii) of a compound of formula (I) according to claim 1 with R being: 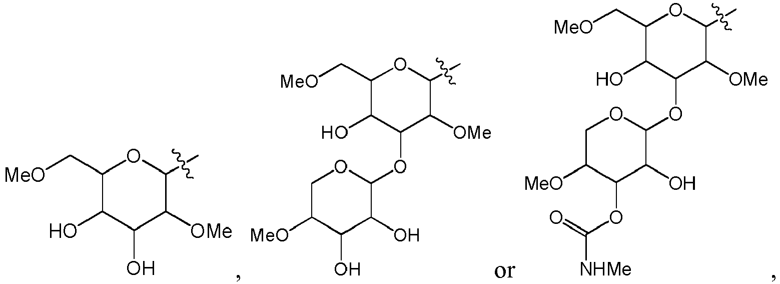
(iv) optionally, hydrolysis of the compound of formula (I) obtained in the preceding step to yield a compound of the following formula (II): 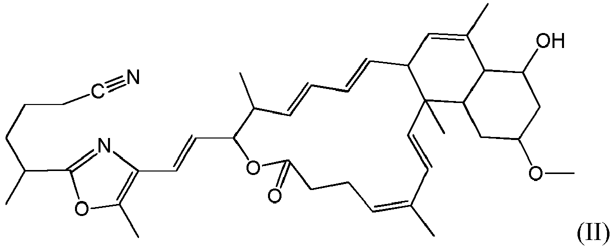 and substitution of the OH functional group of the compound of formula (II) to yield a compound of formula (I) as defined in claim 1, and
(v) optionally, salification, hydration or solvation of the compound of formula (I) obtained in the preceding step (iii) or (iv) to yield a pharmaceutically acceptable salt, hydrate or solvate thereof.

7. The compound according to any one of claims 1 to 6, **characterized in that** it is selected from: 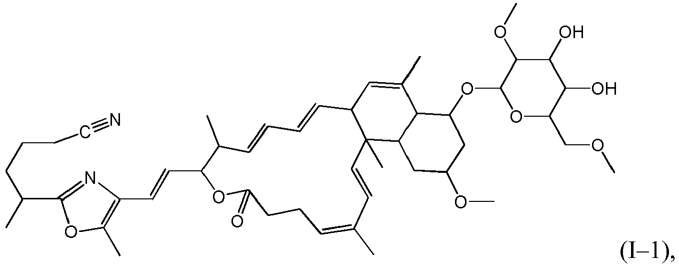 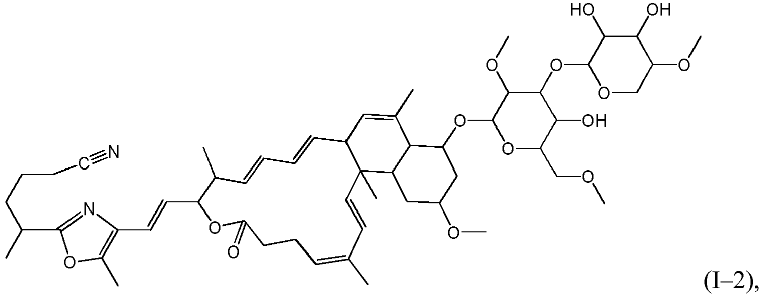 and

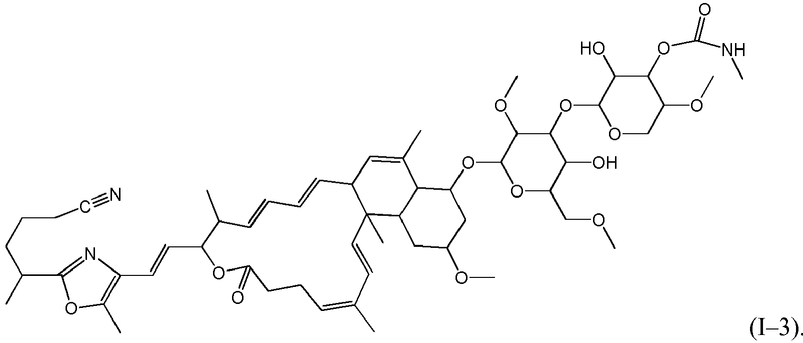

8. The compound according to any one of claims 1 to 7 for use as a medicine.

9. The compound according to any one of claims 1 to 7 for use in the treatment of cancer.

10. A pharmaceutical composition comprising at least one compound of formula (I) according to any one of claims 1 to 7 and a pharmaceutically acceptable excipient.

11. The pharmaceutical composition according to claim 10 for use in the treatment of cancer.

12. A method for the preparation of a compound according to any one of claims 1 to 7, comprising the following successive steps:
(i) one or more macerations of a lyophilizate of a sponge of the family *Verongidae,* such as a *Suberea sp.* sponge, for example *Suberea creba,* with a water/ethanol solution (in particular 10/90 water/ethanol, v/v); followed by filtration to yield a filtrate; then concentration of said filtrate to yield an aqueous syrup,
(ii) addition of water to the aqueous syrup obtained in the preceding step (i), one or more extractions with ethyl acetate and separation of the resulting aqueous and organic phases; concentration of the organic phase or the combined organic phases thus obtained to yield a dry extract,
(iii) isolation from the dry extract obtained in the preceding step (ii) of a compound of formula (I) according to claim 1 with R being: 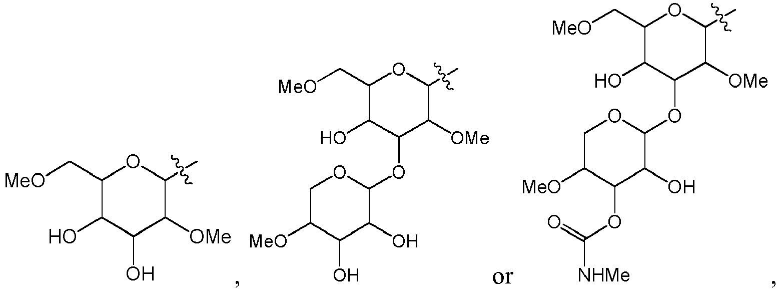
(iv) optionally, hydrolysis of the compound of formula (I) obtained in the preceding step to yield a compound of the following formula (II): 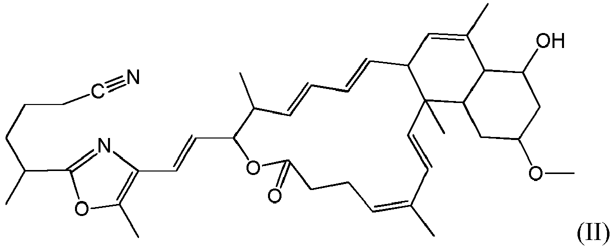 and substitution of the OH functional group of the compound of formula (II) to yield a compound of formula (I) according to claim 1, and
(v) optionally, salification, hydration or solvation of the compound of formula (I) obtained in the preceding step (iii) or (iv) to yield a pharmaceutically acceptable salt, hydrate or solvate thereof.
